# EUROPEAN PATENT APPLICATION

(11) **EP 3 978 623 A1**
(43) Date of publication of application: **06.04.2022**
(21) Application number: 21199197.1
(22) Date of filing: 27.09.2021
(51) Int. Cl.: C12Q 1/6806, C12N 15/10

(54) **VIRUS DETECTION IN WASTEWATER**

(30) Priority: 02.10.2020 US 202063086922 P
(71) Applicant: Luminultra Technologies Ltd., Fredericton, New Brunswick E3B 6G3 (CA)
(72) Inventor: Schmidt, Jordan Jeremy, Windsor, B0N 2T0 (CA); Sharma, Neil Christopher, Gaithersburg, 20878 (US); Huang, Wei, 21042, Ellicott City (US)
(74) Representative: Nederlandsch Octrooibureau

(57) **Abstract**

A method is provided to extract genetic material of a virus, for example the SARS-CoV-2 virus, from a wastewater sample. The wastewater sample is in the range of 0.5 to 5 mL in volume. The wastewater sample may be prefiltered, but is not separated to concentrate the virus in a solids fraction. A lysis buffer is added to the sample, followed by a waiting period of at least one minute, followed by the addition of an alcohol. 1.5-4.5 mg of magnetic beads are added per mL of wastewater sample. The magnetic beads are later separated from the sample. Genetic material is eluted from the magnetic beads, optionally at a temperature of at least 45 °C. A test kit is provided for use in performing the method.

## Description

### FIELD

This specification relates to systems and methods for testing water, for example wastewater including an animal waste stream, for the presence of a virus, for example the SARS-CoV-2 virus.

### BACKGROUND

Various viruses, for example Adenovirus, Norovirus and Enterovirus, are readily transmitted via water. Other viruses, for example the SARS coronavirus ("SARS-CoV") do not survive for extended periods of time in water, but can still be transmitted by water in some circumstances. For example, 300 people were infected with SARS-CoV in a high-rise complex in Hong Kong after the stool of an infected person entered the internal sewer system of the complex, which allowed an aerosol containing the virus to enter the ventilation system. Detecting a virus in water can be used as a way to screen for potential health risks to people exposed to the water.

Detecting a virus in wastewater, whether the virus is viable or not, can also be used as a method to detect or measure the presence of a virus in a group of people that contribute human waste to the wastewater. The virus is carried into the wastewater system through the stools of infected people. For example, wastewater testing has been used to monitor for the presence of poliovirus.

However, the detection and quantification of viruses in wastewater is difficult due to their small size. For example, viruses are too small to be concentrated on standard vacuum filters for analysis. However, concentration is typically required to ensure that a sufficient quantity of the target virus is present in a solution with a volume that is small enough for a viral detection and/or quantification assay. The viral detection and/or quantification assay may be, for example, a molecular assay to detect viral nucleic acids, such as an RT-qPCR assay.

Known methods to concentrate samples generally require one or more of specialized training, specialized expensive equipment, and difficult to obtain or expensive consumables. The techniques also tend to recover a low amount of virus, particularly enveloped viruses such as SARS-CoV-2.

In one example involving the SARS-CoV-2 virus, the KWR Water Research Institute ("KWR") conducted tests for various gene fragments of the novel 2019 coronavirus ("SARS-CoV-2") in samples of sewage entering ten wastewater treatment plants in the Netherlands. No SARS-CoV-2 gene fragments were detected in a first series of samples take on February 5-7, 2020, before the first reported SARS-CoV-2 infection in the Netherlands on February 27, 2020. A second series of samples taken on March 4-5, 2020, showed the presence of one type of SARS-CoV-2 gene fragment in wastewater influent to four of the municipal wastewater treatment plants and in wastewater from the Schiphol Airport. Tests later in March showed the presence of greater amounts, and more types, of SARS-CoV-2 gene fragments in influent to all but one of the wastewater treatment plants in the study. In further study, trends in the number of copies of SARS-CoV-2 in samples from influent to the Amsterdam wastewater treatment plant taken at various times approximately tracked the reported number of COVID-19 hospitalizations in the same city over the same time period. However, the amount of SARS-CoV-2 virus detected in wastewater is believed to lead reported COVID-19 infection trends by about 4-7 days. Wastewater monitoring can thereby provide an early warning of a viral outbreak, a means to determine if a remediation program is effective, or an aggregate measure of the activity of a virus in a population.

In the method used by KWR in the studies described above, samples were sent on ice to a central laboratory. At the laboratory, the testing procedure included centrifuging the samples at 4654xg for 30 minutes to form a pellet containing larger particles such as debris and bacteria. Supernatant (roughly 100 mL) was withdrawn and filtered through a centrifugal ultrafilter with a molecular weight cut-off of 100 kDa at 1500xg for 15 minutes. RNA was extracted from portions of the concentrate (solids fraction) and tested for the SARS-CoV-2 virus by RT-PCR. RNA was extracted from some of the samples using an RNeasy PowerMicrobiome(TM) kit (Qiagen, Hilden, Germany). RNA was extracted from other samples using magnetic extraction reagents from a Biomerieux Nuclisens(TM) kit (Biomerieux, Amersfoort, the Netherlands) in combination with a KingFisher(TM) mL Purification System (Thermo Scientific, Bleiswijk, the Netherlands). The KingFisher(TM) system is an automated magnetic particle processor system that accepts samples of 50 uL to 1 mL in size. Elution of RNA was done in 100 uL of elution buffer in both extraction methods.

### INTRODUCTION

This specification describes alternative systems and methods to prepare a sample of wastewater for use in a viral detection and/or quantification assay.

The system and method use magnetic beads to concentrate viral nucleic acids in the sample directly. That is, while the sample may be treated, for example filtered, to remove bacteria or other contaminants in a solids fraction, the sample is not separated to concentrate the virus in a solids fraction of the sample prior to adding the magnetic beads. The sample may be, for example, 0.5-10 mL or 1-2.5 mL. While this is smaller than a typical sample (usually 40-200 mL), the nucleic acid recovery rate can be sufficient to enable viral detection and/or quantification by way of a molecular genetic assay.

The method includes steps of sample extraction, lysis, binding genetic material onto magnetic beads, separating the magnetic beads with the bound genetic material, and elution of the genetic material from the magnetic beads. The extracted sample may have a volume in the range of 0.5 to 10 mL. In some examples the sample is filtered, with the virus remaining in the liquid fraction, in the sample extraction step. In some examples, at least one lysis buffer is added to the sample at least 1 minute, or at least 5 minutes, before adding an alcohol to the sample. In some examples, the binding step includes adding 1.5 to 4.5 mg of magnetic beads per mL of sample. The method optionally includes washing the magnetic beads in the separation step. In some examples, genetic material is eluted from the magnetic beads at a temperature of at least 45°C. The eluted genetic material may be detected and/or measured, for example, by way of a molecular genetic assay such as RT-qPCR, optionally with primers selected to amplify one or more RNA fragments of the SARS-CoV-2 virus.

### DETAILED DESCRIPTION

In a method described in further detail below, genetic material such as RNA fragments from a virus is extracted from a wastewater sample for further analysis. The wastewater is connected to an animal waste stream. For example, the wastewater may include human sewage. The wastewater may be collected, for example, from the effluent of a building or group of buildings or the influent to a sewage containment or treatment facility such as a wastewater (i.e. sewage) treatment plant.

The extracted genetic material may be detected or quantified by a molecular genetic assay. Examples of molecular assays include reverse transcription polymerase chain reaction (RT-PCR), reverse transcription quantitative polymerase chain reaction RT-qPCR, reverse transcription loop-mediated isothermal amplification (RT-LAMP), transcription-mediated amplification (TMA), clustered regularly interspaced short palindromic repeats (CRISPR) based assays, nucleic acid hybridization using microarray, amplicon-based metagenomic sequencing, or another method. In the case of RT-PCR, RT-qPCR and RT-LAMP, primers are selected for genetic material of a target virus to be detected. Optionally, the target virus may be the SAS-CoV-2 virus.

The method involves the use of nucleic acid extraction beads, alternatively called magnetic beads. These beads are commercially available. The magnetic beads typically have a paramagnetic core, which may be made of an iron oxide such as magnetite. The paramagnetic beads are not attracted to each other in the absence of an external magnetic field. However, the beads but can be separated from a suspension by applying a magnet or other external magnetic field. The core is coated with a coating adapted to reversibly bind to a target of interest. For example, silica coated magnetic beads reversibly bind nucleic acids based on salt concentration.

In an example, the method includes steps of sample extraction, virus lysis, binding genetic material to magnetic beads, separating the magnetic beads from the sample, and elution. In the description below, words such as "buffer", "reagent", "solution" or similar words are used to refer generally to one or more chemical compounds and do not limited to any specific function or form of the compounds unless apparent from the context or explicitly stated. For example, a "buffer" does not necessarily have any pH or other buffering functionality.

### Sample Extraction

In the extraction step, a volume of wastewater is removed from a source. The source contains animal, i.e. human, waste such as feces. The animal population connected to the wastewater is infected, or potentially infected, by one or more target viruses, for example the SARS-CoV-2 virus. The animal waste is typically mixed with one or more other forms of waste such as household greywater, stormwater, commercial wastewater or industrial wastewater.

The wastewater is divided into one or more samples in separate containers. Each sample may have an initial volume in the range of 0.5-10 mL, 0.5-5 mL, or 1-2.5 mL. While a larger sample would be expected to include more of the target virus, the percentage of the original viral genetic material recovered declines with increasing sample volume. Surprisingly, even the absolute amount of viral genetic material recovered can decline with increasing sample volume past an optimal sample volume even though more genetic material is initially present in larger samples. However, a sufficient amount of genetic material can be recovered for a molecular genetic assay such as RT-qPCR even with small samples as describer herein.

Optionally, the extraction step can include filtering the wastewater before the wastewater is divided into the samples. The filter may have a pore size of 1 um or less. The filter may separate bacteria and some suspended solids into a solids fraction of the wastewater. Virus in the wastewater passes through the filter and remains in a liquid fraction of the wastewater.

### Virus Lysis

In the lysis step, one or more lysis buffers are mixed with the sample to form a first mixture followed by an incubation period. The lysis buffer may contain one or more enzymes and/or chaotropic agents. Useful enzymes include, for example, Proteinase K. Useful chaotropic agents include, for example, guanidinium thiocyanate, guanidinium chloride and alcohols such as ethanol or isopropanol. However, the use of an alcohol is preferably delayed until after the incubation period of the lysis step. Optionally, the first mixture may include one or more surfactants, for example Triton X-100. Optionally, the first mixture may include a binding enhancer such as carrier RNA, for example poly(A) RNA (poly(A) potassium salt, prepared from ADP with polynucleotide phosphorylase). In some examples, the first mixture includes a guanidinium salt, Proteinase K, and carrier RNA.

In the incubation period, the first mixture is maintained for a period of time at one or more specified temperatures. The period of time may be, for example, at least 1 minute, at least 5 minutes, or at least 10 minutes. The specified temperature(s) may be, for example, at least 25°C or at least 30°C.

### Binding Genetic Material to Magnetic Beads

In the binding step, the incubated first mixture is mixed with magnetic beads and an alcohol to form a second mixture, followed by another incubation period. The magnetic beads may have, for example, a silica-based coating. To facilitate mixing small amounts of the magnetic beads, the magnetic beads may be first suspended in water. The concentration of magnetic beads in the suspension may be, for example, 10-100 g/L or 20-50 g/L. A small volume of the magnetic bead suspension, for example 20% or less or 10% or less of the initial volume of the wastewater sample, is mixed with the sample. The second mixture may contain 1.5-4.5 mg of magnetic beads per mL of the initial volume of the wastewater sample.

The alcohol may be, for example, ethanol or isopropanol. Optionally, the second mixture contains 2-5 mL of 100% ethanol per mL of initial volume of the wastewater sample.

In the incubation period, the second mixture is maintained for a period of time at one or more specified temperatures. The period of time may be, for example, at least 1 minute, at least 5 minutes, or at least 10 minutes. The temperature(s) may be, for example, at least 25°C or at least 30°C.

### Separating Magnetic Beads from the Sample

The magnetic beads may be separated from the sample (i.e. the second mixture), for example, by holding a magnet against a sample container while pouring off the liquid in the sample container.

The separation step may also include washing the magnetic beads. For example, a wash buffer may be added to the sample container and mixed with the magnetic beads. The magnetic beads are then separated from the wash buffer, for example, by holding a magnet against a sample container while pouring off the liquid in the sample container. These steps may be repeated, for example 1 to 10 times, with the same or a different wash buffer. A wash buffer, or a series of wash buffers, may contain for example one or more of guanidinium chloride, guanidinium thiocyanate, ethanol, isopropanol and water.

### Elution

In the elution step, the magnetic beads are mixed with an elution buffer to form a third mixture, followed by an incubation period. The elution buffer may contain, for example, Tris-HCI and/or EDTA.

In the incubation period, the third mixture is maintained for a period of time at one or more specified temperatures. The period of time may be, for example, at least 1 minute, at least 5 minutes, or at least 10 minutes. The temperature(s) may be, for example, at least 30°C, at least 45°C or at least 60°C.

After the incubation period, the elution buffer contains extracted genetic material of the target virus. For example, the elution buffer may contain RNA fragments of the SARS-CoV-2 virus. The elution buffer is preferably separated from the magnetic beads before analysis of the elution buffer to detect or quantify the viral genetic material. The magnetic beads may be separated from the elution buffer, for example, by holding a magnet against the sample container while pouring off the elution buffer into another container.

The total time required to extract the genetic material from the sample may be, for example, 150 minutes or less or 60 minutes or less. The elution buffer in the second container may be combined with one or more molecular assay reagents and placed into one or more containers adapted for use in the molecular assay device. For example, the elution buffer may be mixed with primers and divided into one or more sample tubes for use in an RT-qPCR or RT-LAMP testing device.

### Test Kit

A test kit for use with a method as described above may include: one or more lysis buffers comprising guanidinium thiocyanate, Proteinase K and carrier RNA; silica-coated magnetic beads; a first wash buffer containing guanidinium thiocyanate and ethanol, a second wash buffer containing ethanol; and, an elution buffer, optionally containing Tri-HCI and/or EDTA. The test kit may also contain suitable liquid handling devices such as containers and pipettes.

### Example 1: SARS-CoV-2 RNA Extraction from Municipal Wastewater using Magnetic Beads

### Sample collection and pre-treatment

1. Collect a 1 L sample of primary influent or raw wastewater (grab sample or composite) in a sterile container. Maintain the sample at 4 °C during transportation and handling in the lab or before testing on site.
2. Aliquot 50-mL of wastewater sample for pre-treatment (filtration). Store the remaining sample at -20 or -80 °C for future analyses.
3. Before filtration, sterilize the funnel top, membrane support and filtration flask by autoclaving.
4. Assemble a vacuum filtration dispositive with Mixed Cellulose Ester Membranes (0.45 µm pore size) for each sample that will be processed. Use proper aseptic techniques when handling filters and filtration units. All sample processing within a lab should be carried out within a Class II Biosafety Cabinet.
5. Slowly add the 50-mL of wastewater to the filtration funnel top. Turn on the vacuum to start the filtration. Check for leakages and ensure all the sample has been filtered. Depending on the turbidity, the filtration takes up to 30 minutes.
6. After filtration, use sterile tweezers to remove the filter. Collect the filtered wastewater in a sterile 50-mL conical tube.

### RNA extraction

1. In a 15-mL sterile conical tube, add 6 mL of Lysis Buffer concentrate (guanidinium thiocyate and Triton X-100), 514 µL of Lysis/Binding Enhancer (Proteinase K solution), 17.1 µL of Carrier RNA and 1 mL of filtered wastewater.
2. Vortex the tube for 30 s (3000 rpm) and incubate the mixture at 30 °C for 10 min.
3. After incubation, add 3.5 mL of 100% Ethanol and mix thorough by gently inverting the tube 5 times.
4. Add 100 µL of 20 g/L silica-coated magnetic bead suspension (briefly vortex before use) to the tube and vortex for 30 s (3000 rpm). Incubate again at 30 °C for 10 min.
5. After incubation, precipitate the magnetic beads by applying a magnet. Discard the supernatant.
6. Transfer the magnetic beads into a 2-mL microcentrifuge tube by resuspending them in 1 mL of first wash I solution (guanidinium thiocyanate and ethanol solution). Vortex the microtube for 30 s (1500 rpm).
7. Apply the magnet to precipitate the magnetic beads. Pour off the supernatant.
8. Repeat steps 6 and 7 two times more for a total of three washes with first wash solution.
9. Add 1 mL of second wash solution (diluted ethanol) to the magnetic beads. Vortex for 30 s (1500 rpm).
10. Apply the magnet to precipitate the magnetic beads. Discard the supernatant.
11. Perform steps 9 and 10 two times more for a total of three washes with second wash solution.
12. Place the microtubes containing the magnetic beads in a rack with the caps off to evaporate the residual ethanol at room temperature. Ensure all the ethanol is evaporated before proceeding to the next step. The process takes up to 1 h at room temperature. Optionally, apply mild heat, air flow or vacuum to reduce the evaporation time.
13. Add 50 µL of elution buffer (Tris-HCL and EDTA solution) preheated at 60 °C to the magnetic beads. Vortex for 30 s (1500 rpm). Ensure the magnetic beads are resuspended in the elution buffer.
14. Incubate the magnetic beads at 60 °C for 5 minutes.
15. After incubation, apply the magnet and separate the magnetic beads from the elution buffer.
16. Collect the eluted RNA into a new sterile 2-mL tube for downstream analysis.
17. Store the RNA at -80 °C until further analysis.

### Example 2: Contemporaneous lysis and addition of magnetic beads

SARS-CoV-2 virus genetic material was separated from samples of wastewater using a method similar to the method described in relation to Example 1, but with three variations. Firstly, an incubation period was not provided prior to the addition of the magnetic beads. That is, the lysis buffer, ethanol and magnetic beads were added contemporaneously. Secondly, the samples were not pre-filtered. Thirdly, the elution temperature was 30 degrees C.

Trials varied in volume and in the amount of magnetic beads that were added. The test parameters in the trials and virus recovery percentage are given in Table 1. The magnetic bead mixture had a solids concentration of 20 g of magnetic beads per mL of water prior to being added to the wastewater sample. Each sample was spiked to the same initial concentration of a SARS-CoV-2 reference material with a viral protein coat (positive reference material from AccuPlex(TM) SARS-CoV-2 Referance Material Kit from SeraCare). Percentage recovery is based on the amount of reference material initially added, i.e. a 10% virus recovery indicates that downstream RT-qPCR analysis indicated a sample concentration of 10% of the reference material concentration initially added to the sample.

**Table 1**

| Initial Volume of Wastewater Sample (mL) | Volume of Magnetic Bead Mixture (uL) added per mL of Sample | Elution Temperature (°C) | DTT concentration (mM) | Pre-filtration | Virus Recovery (%) |
|---|---|---|---|---|---|
| 1 | 30 | 30 | 0 | No | 2.40 |
| 2.35 | 30 | 30 | 0 | No | 0.96 |
| 1 | 50 | 30 | 0 | No | 0.66 |
| 2.35 | 50 | 30 | 0 | No | 0.30 |
| 2.8 | 20 | 30 | 0 | No | 0.25 |

As indicated in Table 1, the percentage of virus recovery was low in all trials.

### Example 3: Delayed addition of magnetic beads

SARS-CoV-2 virus genetic material was separated from samples of wastewater using a method similar to the method described in relation to Example 1, but with two variations. Firstly, the samples were not pre-filtered. Secondly, the elution temperature was 30 degrees C for some trials. Unlike Example 2, an incubation period of 10 minutes was provided between the addition of the lysis buffer and the addition of ethanol. Magnetic beads were added with no material delay after the addition of the ethanol.

Trials varied in volume, in the amount of magnetic beads that were added, and in elution temperature. The test parameters in the trials and virus recovery percentage are given in Table 2. The magnetic bead mixture had a solids concentration of 20 g of magnetic beads per mL of water prior to being added to the wastewater sample.

**Table 2**

| Initial Volume of Wastewater Sample (mL) | Volume of Magnetic Bead Mixture (uL) added per mL of Sample | Elution Temperature (oC) | DTT concentration (mM) | Pre-filtration | Virus Recovery (%) |
|---|---|---|---|---|---|
| 1 | 15 | 30 | 0 | No | 2.7 |
| 2.5 | 15 | 30 | 0 | No | 1.3 |
| 1 | 100 | 30 | 0 | No | 6.2 |
| 2.5 | 100 | 30 | 0 | No | 2.3 |
| 1 | 15 | 60 | 0 | No | 5.6 |
| 2.5 | 15 | 60 | 0 | No | 4.0 |
| 1 | 100 | 60 | 0 | No | 11.8 |
| 2.5 | 100 | 60 | 0 | No | 4.4 |

As indicated in Table 2, the percentage of virus recovered was higher for the 1 mL wastewater samples than for the 2.5 mL wastewater samples. Because the volume of the 2.5 mL sample is larger, the mass recovered is similar in both samples. Accordingly, the 2.5 L sample is a useful sample size, but the 1 mL sample provides a similar mass of target material recovered while using less magnetic beads.

Table 2 further indicates that the percentage of virus recovered was higher when 100 uL of magnetic bead mixture was added per mL of wastewater sample than when 15 uL of magnetic bead mixture was added per mL of wastewater sample. The percentage of virus recovered was higher with a 60°C elution temperature than with a 30°C elution temperature.

Considering the data in Table 2 as a whole relative to the data in Table 1, the addition of an incubation period after adding the lysis buffer appeared to increase the percentage of virus recovered.

### Example 4: Addition of DTT and prefiltering the wastewater

SARS-CoV-2 genetic material was separated from samples of wastewater using a method similar to the method described in relation to Example 1, but with two variations. Firstly, some of the samples were not pre-filtered. Secondly, dithiothreitol (DTT), a redox agent, was added in some of the trials with the lysis buffer. An incubation period of 10 minutes was provided between the addition of the lysis buffer and the addition of ethanol. Magnetic beads were added with no material delay after the addition of the ethanol.

The test parameters in the trials and virus recovery percentage are given in Table 3. The magnetic bead mixture had a solids concentration of 20 g of magnetic beads per mL of water prior to being added to the wastewater sample.

**Table 3**

| Initial Volume of Wastewater Sample (mL) | Volume of Magnetic Bead Mixture (uL) added per mL of Sample | Elution Temperature (oC) | DTT concentration (mM) | Pre-filtration | Virus Recovery (%) |
|---|---|---|---|---|---|
| 1 | 100 | 60 | 0 | No | 17.00 |
| 1 | 100 | 60 | 20 | No | 13.67 |
| 1 | 100 | 60 | 0 | Yes | 18.67 |
| 1 | 100 | 60 | 20 | Yes | 15.00 |

As indicated in Table 2, adding DTT did not improve the percentage of virus recovered. The percentage of virus recovered was higher when the wastewater samples were prefiltered.

Considering the data in Table 3 as a whole relative, the percentage of virus recovered was very high in all of the trials.

## Claims

1. A method for extracting genetic material of a virus from wastewater comprising an animal waste stream, the method comprising the steps of,
obtaining a sample of the wastewater;
mixing one or more lysis buffers and 0.5 to 5 mL of the sample to obtain a first mixture;
adding an alcohol to the first mixture after incubating the first mixture for at least one minute, and adding magnetic beads to the first mixture, to obtain a second mixture;
incubating the second mixture for at least one minute;
separating the magnetic beads from the second mixture;
mixing the separated magnetic beads with an elution buffer to obtain a third mixture; and,
incubating the third mixture for at least one minute.

2. The method of claim 1 wherein the alcohol comprises ethanol.

3. The method of claim 1 or 2 comprising separating the separated magnetic beads from the elution buffer.

4. The method of any of claims 1 to 3 comprising performing a molecular genetic assay on the elution buffer.

5. The method of claim 4 wherein the molecular assay comprises RT-qPCR.

6. The method of claim 4 or 5 wherein the molecular genetic assay comprises primers for one or more RNA fragments of the SARS-CoV-2 virus.

7. The method of any of claims 1 to 6 wherein the third mixture is incubated at a temperature of at least 45 °C.

8. The method of any of claims 1 to 7 wherein the sample is filtered before mixing with the lysis buffer, but not separated to concentrate the virus in a solids fraction.

9. The method of any of claims 1 to 8 wherein the wastewater comprises a human waste stream.

10. The method of any of claims 1 to 9 comprising washing the separated magnetic beads.

11. The method of any of claims 1 to 10 comprising adding 1.5-4.5 mg magnetic beads per mL of wastewater.

12. The method of any of claims 1 to 11 wherein the wastewater comprises the SARS-CoV-2 virus.

13. The method of any of claims 1 to 12 wherein the sample is obtained from influent to a sewage treatment facility or effluent sewage from a building or group of buildings.

14. A test kit for use in performing the method.

15. The test kit of claim 14 comprising,
a lysis buffer comprising guanidinium thiocyanate and Triton X-100;
a lysis/binding enhancer comprising Proteinase K;
carrier RNA;
silica-coated magnetic beads;
a first wash buffer containing guanidinium thiocyanate and ethanol;
a second wash buffer containing ethanol; and,
an elution buffer, optionally containing Tri-HCI and/or EDTA.
